(19) Europäisches Patentamt
European Patent Office
Office européen des brevets

(11) Publication number: 0 156 495
A1

(12) # EUROPEAN PATENT APPLICATION

(21) Application number: 85301113.8

(22) Date of filing: 20.02.85

(51) Int. Cl.⁴: C 07 D 213/73
//C07D213/74

(30) Priority: 21.02.84 US 582198

(43) Date of publication of application: 02.10.85 Bulletin 85/40

(84) Designated Contracting States: AT BE CH DE FR GB IT LI NL SE

(71) Applicant: ELI LILLY AND COMPANY
Lilly Corporate Center
Indianapolis Indiana 46285(US)

(72) Inventor: Campbell, Jack Beuford
1809 South Chester
Indianapolis Indiana 46203(US)

(72) Inventor: Greene, James Michael
607 West Ralston Road
Indianapolis Indiana 46217(US)

(72) Inventor: Lavagnino, Edward Ralph
4010 Rommel Drive
Indianapolis Indiana 46208(US)

(72) Inventor: Pike, Andrew Joseph
5226 Cornelius Avenue
Indianapolis Indiana 46208(US)

(74) Representative: Tapping, Kenneth George et al,
Lilly Industries Limited Patent Department Erl Wood Manor
Windlesham Surrey, GU20 6PH(GB)

(54) **Diaminopyridines via hydrogenation of nitrobenzylaminopyridines.**

(57) Diaminopyridines are prepared by hydrogenating the corresponding nitrobenzylaminopyridines in the presence of a palladium catalyst.

EP 0 156 495 A1

Croydon Printing Company Ltd.

# DIAMINOPYRIDINES VIA HYDROGENATION OF NITROBENZYLAMINOPYRIDINES

The present invention relates to a process for preparing a diaminopyridine of the formula

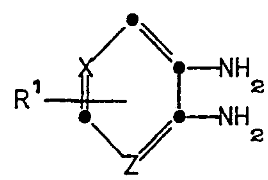

or a salt thereof, wherein $R^1$ is hydrogen or $C_1$-$C_4$ alkyl and one of X and Z is CH and the other is N, comprising reacting a nitrobenzylaminopyridine of the formula

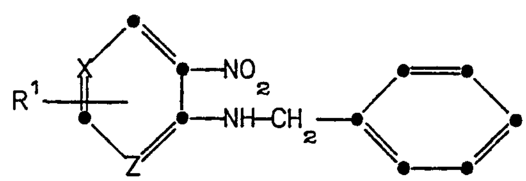

or a salt thereof, with hydrogen in the presence of a catalytically sufficient amount of a palladium catalyst and a suitable solvent at a temperature in the range of from about 50°C to about 150°C.

Temperature will be expressed herein as degrees Celsius.

In the above formula, $C_1$-$C_4$ alkyl represents a straight or branched alkyl chain having from one to four

carbon atoms. Typical $C_1$-$C_4$ alkyl groups include methyl, ethyl, n-propyl, isopropyl, n-butyl, isobutyl, sec-butyl and t-butyl.

Typical salts comprehended herein include the acid addition salts, such as hydriodic, hydrobromic and especially hydrochloric acid salts.

While the entire scope of process variables taught herein are believed operable, the present process does have preferred aspects. In the above formula, preferably $R^1$ is hydrogen and X is N and Z is CH. Other preferred process conditions will be noted hereinafter.

The process of the present invention is generally conducted as follows. Approximately one equivalent of the nitrobenzylaminopyridine is dissolved in a suitable solvent such as any one of the several alcohols, for example, methanol or preferably ethanol; the saturated hydrocarbons, such as pentane or hexane; the aromatic hydrocarbons such as benzene, toluene or the xylenes; the ethers, such as diethyl ether or tetrahydrofuran; and other similar non-reactive organic solvents. Of these, the alcohols are preferred. The amount of solvent employed herein is not critical, but typically a quantity sufficient to dissolve the nitrobenzylaminopyridine starting material and expected product is employed. Mixtures of solvents may be used as well.

The nitrobenzylaminopyridine solution is then combined with a catalytically sufficient amount of a palladium catalyst. The term "catalytically sufficient amount", as defined herein, refers to an amount of

palladium catalyst which modifies, and especially increases, the rate of the chemical reaction without being consumed in the process. This amount will generally be from about 0.1 to 1.5 equivalents of palladium catalyst by weight to weight of starting nitrobenzylaminopyridine, more preferably at a weight equal to the weight of the starting material. Exemplary palladium catalysts for use herein include palladium oxide, palladium on calcium carbonate and especially palladium on carbon.

The reaction is conducted in the presence of hydrogen gas and is typically carried out until the theoretical amount of four equivalents of hydrogen uptake is observed.

The reaction is conducted at a temperature in the range of from about 50°C to about 150°C, more preferably from about 50°C to about 100°C, typically for a period of time in the range of 4 to 48 hours.

The diaminopyridine thus prepared may be isolated by procedures well known in the art. Typically the reaction mixture is filtered through Celite and the filtrate is evaporated to dryness under reduced pressure The product thus isolated may be further purified if desired by standard procedures such as crystallization from common solvents or purification over solid supports such as silica gel or alumina.

The present process has been found to produce large quantities of either 2,3- or 3,4-diaminopyridines in high yields and to provide the product consistently in high purity, so that the compound may be used in the

preparation of biologically active compounds without additional expensive purification steps.

The diaminopyridine compounds prepared by the present process are preferably used as intermediates in the synthesis of a variety of compounds, for example pharmaceuticals useful for the treatment of a variety of human disorders. See, e.g. U.S. Patent Nos. 3,985,891 and 4,327,100 (use of 2,3-diaminopyridine in the preparation of imidazo[4,5-b]pyridine inotropic agents); and European Patent Application 72,926 (use of 3,4-diaminopyridine in the preparation of imidazo[4,5-c]pyridine inotropic agents). The reader is also referred to U.S. Patent No. 4,386,095, which teaches the use of diaminopyridines to ameliorate decreased cognition occurring in ageing mammals.

The nitrobenzylaminopyridine starting materials employed in the present process are prepared by procedures well known in the art, or by processes analogous to such prior art procedures. Generally, either a nitrochloropyridine or $C_1$-$C_4$ alkoxynitropyridine derivative is reacted with benzylamine under standard amination conditions. See, e.g. Bremer in Ann. 518, 274-89 (1935) for the synthesis of 3-nitro-4-benzylaminopyridine from the corresponding 3-nitro-4-chloropyridine derivative.

The following Examples further illustrate specific aspects of the present process.

## Example 1

### 3,4-Diaminopyridine

A mixture of 5.7 g (0.025 mol.) of 3-nitro-4-benzylaminopyridine, 5.7 g of 5 percent palladium on carbon in 90 ml of ethanol was hydrogenated at 80°C for 30 hours at an initial pressure of 60 psi. The reaction mixture was filtered through Celite and the filtrate was concentrated under reduced pressure. The residue was crystallized from ethyl acetate/methanol to afford 2.07 g of 3,4-diaminopyridine. Yield 76%. mp = 199°-201°C.

## Example 2

### 2,3-Diaminopyridine

A mixture of 4.6 g (0.02 mol) of 3-nitro-2-benzylaminopyridine and 4.6 g of 5 percent palladium on carbon in 90 ml of ethanol was hydrogenated at 70°-80°C for approximately 16 hours at an initial pressure of 60 psi. The reaction mixture was filtered through Celite and the filtrate was evaporated to dryness under reduced pressure. The residue was crystallized from ethyl acetate to afford 2,3-diaminopyridine. mp = 112°-114°C. m/e 109. The presence of the product was also verified by a thin layer chromatographic comparison of the product with an authentic reference sample.

## CLAIMS

1. A process for preparing a diaminopyridine of the formula

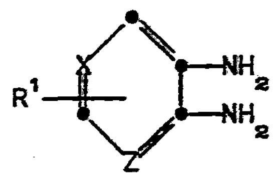

or a salt thereof, wherein $R^1$ is hydrogen or $C_1$-$C_4$ alkyl and one of X and Z is CH and the other is N, comprising reacting a nitrobenzylaminopyridine of the formula

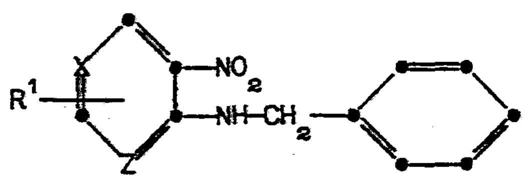

or a salt thereof, with hydrogen in the presence of a catalytically sufficient amount of a palladium catalyst and a suitable solvent at a temperature in the range of from about 50°C to about 150°C.

2. A process of Claim 1 wherein $R^1$ is hydrogen.

3. A process of Claim 2 wherein X is N and Z is CH.

4. A process of Claim 3 wherein the suitable solvent is ethanol.

5. A process of Claim 3 wherein the palladium catalyst is palladium on carbon.

6. A process of Claim 2 wherein X is CH and Z is N.

7. A process of Claim 6 wherein the suitable solvent is ethanol.

8. A process of Claim 6 wherein the palladium catalyst is palladium on carbon.

9. A process of Claim 1 wherein the reaction is conducted at a temperature in the range of from about 50°C to about 100°C.

European Patent Office

# EUROPEAN SEARCH REPORT

0156495
Application number

EP 85 30 1113

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.4) |
|---|---|---|---|
| Y | DE-A-1 670 522 (DEGUSSA)<br>* Page 3, lines 1-8; examples 1,8 *<br>--- | 1-9 | C 07 D 213/73 //<br>C 07 D 213/74 |
| Y | GB-A- 870 027 (GAF CORP.)<br>* Example 5 *<br>--- | 1-9 | |
| Y | CHEMICAL ABSTRACTS, vol. 55, no. 17, 21st August 1961, Columbus, Ohio, USA; K.H. TAFFS et al. "Preparation and oxidation of some bisbenzimidazoles and benzimidazolylhydroxypropionic acids", abstract no. 16520i-16522d, & J. ORG. CHEM. no. 26, 1961, pages 462-467<br>* Column 16521f *<br>--- | 1-9 | TECHNICAL FIELDS SEARCHED (Int. Cl.4) |
| A | HOUBEN-WEYL "Methoden der organischen Chemie" 4th edition 1957, GEORG THIEME VERLAG, Stuttgart F. MÖLLER, "Amine durch Spaltung", pages 968-973<br>--- | | C 07 D 213/00 |
| A | HOUBEN-WEYL "Methoden der organischen Chemie" 4th edition/1c, reduction I 1980, GEORG THIEME VERLAG, Stuttgart E. REIMANN "Katalytische Hydrierung", pages 400-404<br>----- | | |

The present search report has been drawn up for all claims

| Place of search | Date of completion of the search | Examiner |
|---|---|---|
| BERLIN | 03-05-1985 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document